(19) Europäisches Patentamt
European Patent Office
Office européen des brevets

(11) Numéro de publication : **0 318 392 B1**

# (12) FASCICULE DE BREVET EUROPEEN

(45) Date de publication du fascicule du brevet : **15.04.92 Bulletin 92/16**

(51) Int. Cl.$^5$ : **C07F 9/547,** A61K 31/675

(21) Numéro de dépôt : **88402967.9**

(22) Date de dépôt : **25.11.88**

(54) **Nouveaux dérivés N-(vinblastinoyl-23) d'acide amino-1 méthylphosphonique, leurs procédés de préparation et les compositions pharmaceutiques qui les contiennent.**

(30) Priorité : **25.11.87 FR 8716327**

(43) Date de publication de la demande : **31.05.89 Bulletin 89/22**

(45) Mention de la délivrance du brevet : **15.04.92 Bulletin 92/16**

(84) Etats contractants désignés : **AT BE CH DE ES FR GB GR IT LI LU NL SE**

(56) Documents cités :
**WO-A-83/00486**
**FR-A- 2 366 020**

(73) Titulaire : **ADIR ET COMPAGNIE**
**1 rue Carle Hébert**
**F-92415 Courbevoie Cédex (FR)**

(72) Inventeur : **Lavielle, Gilbert**
**1 Avenue Lilly**
**F-78170 La Celle Saint-Cloud (FR)**
Inventeur : **Hautefaye, Patrick**
**9 Rue du Pré aux Moutons**
**F-77170 Sevron Brie Comte Robert (FR)**
Inventeur : **Cudennec, Claude**
**22 Bis Avenue de Circourt**
**F-78170 La Celle Saint-Cloud (FR)**

**EP 0 318 392 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux dérivés N-(vinblastinoyl-23) d'acide amino-1 méthyl phosphonique, leurs procédés de préparation et les compositions pharmaceutiques qui les renferment.

Les alcaloïdes bisindoliques du type de la vinblastine, et certains de leurs dérivés obtenus par transformation chimique des alcaloïdes naturels, sont connus dans la littérature et utilisés en thérapeutique principalement en chimiothérapie anticancéreuse. (Brevets américains 3,097,137 ; 3,205,220, et Brevets belges 659,112 ; 813,168).

En effet, des composés tels que la vinblastine, la vincristine et la vindésine trouvent leurs applications pour le traitement des leucémies et certaines tumeurs solides. Toutefois, ces composés possèdent une toxicité élevée.

Dans le but d'obtenir des composés ayant une toxicité moindre et une activité antitumorale plus importante, certains dérivés N-(désacétyl-0-4 vinblastinoyl-23) aminés tels que la vindésine, ont été préparés (Brevet belge 813.168). Plus récemment, il a été constaté que des dérivés N-(vinblastinoyl-23) d'acides aminés ou de peptides, et plus spécialement le vintryptol, sont doués d'une activité très intéressante sur la leucémie L 1210 et sur les tumeurs expérimentales P 388, et ont une moindre toxicité (Demande Européenne 041.935). Certains dérivés de vinblastine et d'acide thiophosphonique sont aussi connus. (Demande Française N°77.19877 et Demande Européenne 083.600).

La demanderesse a maintenant découvert que certains dérivés N-(vinblastinoyl-23) d'acide amino-1 méthylphosphonique, de structure originale, possèdent des propriétés pharmacologiques très intéressantes. En effet, les composés de la présente invention sont doués d'une activité antitumorale très supérieure à tous les dérivés de vinblastine connus. En plus, les toxicités observées sont significativement inférieures à celles des produits de référence.

La présente invention a plus particulièrement pour objet les dérivés N-(vinblastinoyl-23) d'acide amino-1 méthyl phosphonique de formule générale I :

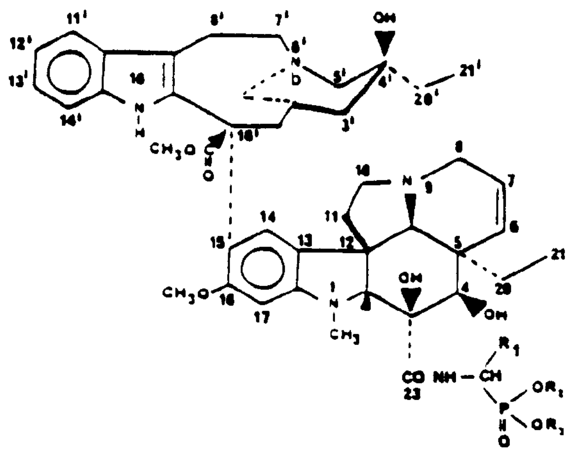

(I)

dans laquelle :

- $R_1$ représente un atome d'hydrogène, un radical alkyle linéaire ou ramifié renfermant de 1 à 6 atomes de carbone, un radical alkylène linéaire ou ramifié renfermant de 1 à 6 atomes de carbone, un radical arylalkyle de 7 à 10 atomes de carbone pouvant porter comme substituant sur le cycle aromatique un atome d'halogène, un radical hydroxyle ou un radical alkyle ou alcoxy renfermant chacun de 1 à 5 atomes de carbone, un radical indolyl-2 méthyle, un radical imidazolyl-4 méthyle, ou un radical alcoxycarbonylméthyle renfermant de 3 à 11 atomes de carbone,
- $R_2$ et $R_3$ identiques ou différents représentent chacun un radical alkyle linéaire ou ramifié renfermant de 1 à 4 atomes de carbone,

sous forme de mélange de diastéréo-isomères ou d'isomères purs, leurs $N_{b'}$-oxydes et leurs sels d'addition à un acide minéral ou organique pharmaceutiquement acceptable.

La présente invention a également pour objet le procédé de préparation de composés de formule générale I, caractérisé en ce que l'on fait réagir une amine, sous forme racémique ou optiquement pure, de formule générale II :

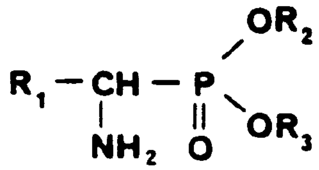

(II)

dans laquelle la définition de $R_1$, $R_2$, et $R_3$ demeure celle définie précédemment pour la formule générale I,

avec le descarbométhoxy-3 désacétyl-0-4 vinblastine carboxazide-3 de formule III,

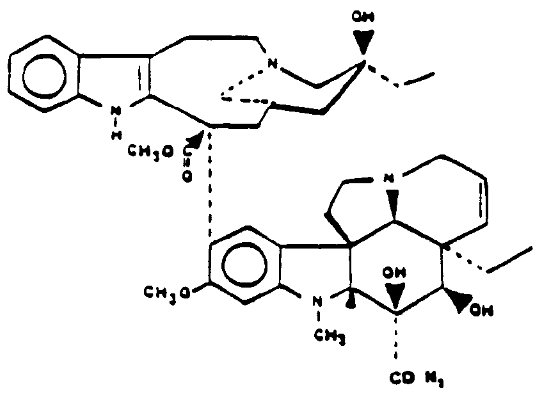

(III)

pour former respectivement sous forme de mélange de diastéréo-isomères ou d'isomères purs les composés de formule générale I,

lesquels ensuite on peut :

– soit salifier avec un acide minéral ou organique pharmaceutiquement acceptable.

– soit transformer aux $N_{b'}$-oxydes correspondants à l'aide d'un solvant organique basique saturé d'oxygène.

Les amino-1 méthyl phosphonates, composés de formule générale II, peuvent être préparés selon trois procédés :

– soit par réduction à l'aide de zinc des composés de formule générale IV :

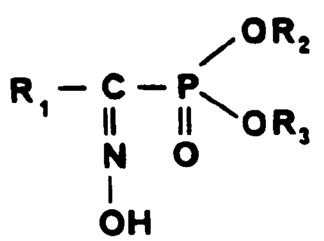

(IV)

dans laquelle $R_1$, $R_2$ et $R_3$ ont la signification précédemment définie pour la formule I, en solution dans l'acide formique ;

– soit par alkylation des imines de formule générale V :

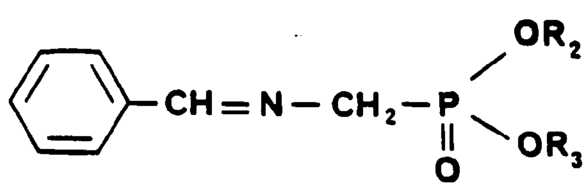

(V)

dans laquelle $R_2$ et $R_3$ ont la signification précédemment définie pour la formule I,

à l'aide d'un halogénure d'alkyle de formule générale VI,

$R_1X$ (VI)

dans laquelle la définition de $R_1$ reste identique à celle donnée pour la formule I selon la méthode décrite dans Bull.Soc.Chim.Fr.(1978),II, p.95 ;

– soit par action du diphénylphosphoryle azide (D.P.P.A.) sur des acides de formule générale VII,

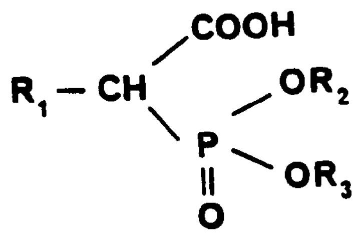

(VII)

dans laquelle $R_1$, $R_2$, $R_3$ ont la signification précédemment définie pour la formule I,

pour former les carbamates de formule générale VIII,

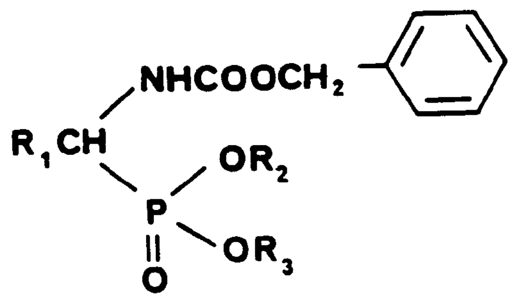

(VIII)

dans laquelle la définition de $R_1$, $R_2$ et $R_3$ reste identique à celle donnée pour la formule I,

qui sont soumis ensuite à une hydrogénolyse catalytique pour former les amines de formule générale II (Tetrahedron Letters,(1983),24,(49), p.5461).

Les composés de formule générale IV sont obtenues par action de l'hydroxylamine sur les cétonnes de formule générale IX,

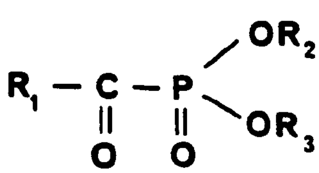

(IX)

dans laquelle $R_1$, $R_2$ et $R_3$ ont la signification précédemment définie pour la formule I, selon le procédé décrit dans Synthesis,(1981),p.57. La préparation des composés de formule générale IX est connue (Houben Weyl, Methoden der Organischen Chemie, Georg Thiene Verlag, Stuttgart, 5e ed, vol. 12/1,p.453).

La préparation des composés de formule générale V est décrite dans Tetrahedron Letters,(1973),46,p.4645.

Le descarbométhoxy-3 désacétyl-0-4 vinblastine carboxazide-3, composé de formule III est préparé en deux étapes. (J.Med.Chem.,(1978),21, p.88). La première étape consiste à additionner un excès d'hydrazine anhydre à une solution de vinblastine base dans le méthanol anhydre. Le composé obtenu, de formule X,

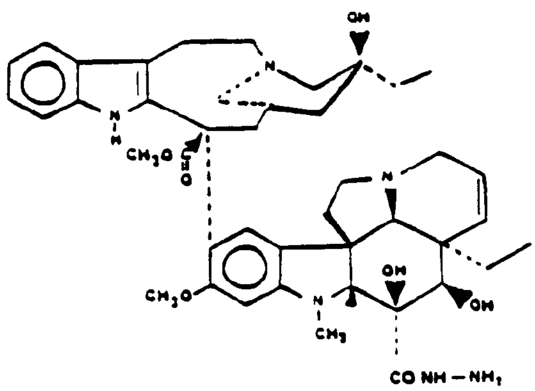

(X)

est ensuite soumis à l'action du nitrite de sodium en milieu acide, pour former le descarbométhoxy-3 désacétyl-0-4 vinblastine carboxazide-3, composé de formule III.

L'acide utilisé lors de cette dernière réaction peut être l'acide chlorhydrique. La température du milieu réactionnel est maintenue entre 0°-5°C.

Le descarbométhoxy-3 désacétyl-0-4 vinblastine carboxazide-3 formé est extrait ensuite par un solvant aprotique non soluble à l'eau de préférence le chlorure de méthylène. Le composé de formule III n'est de préférence pas isolé. En effet, la solution organique le contenant est concentrée, et ensuite le composé de formule III est mis en contact à température ambiante avec les dérivés d'acide amino-1 méthyl phosphonique de formule générale II.

Les amines de formule générale II, peuvent être obtenues optiquement pures ou par cristallisation fractionnée de leurs sels avec un acide optiquement pur, (J.Org.Chem.,(1963),28,p.2483) ou selon le procédé décrit dans Liebigs Ann.Chem.,(1987),p.45.

Les composés de formule générale I peuvent être aussi obtenus sous forme de diastéréo-isomères purs par condensation du descarbométhoxy-3 désacétyl-0-4 vinblastine carboxazide-3 avec une amine de formule générale II optiquement pure ou à partir de mélange de diastéréo-isomères, que l'on sépare ensuite par chromatographie liquide haute pression (H.P.L.C.).

Les composés de la formule générale I sont des dérivés de descarbométhoxy-3 désacétyl-0-4 vinblastine carboxamide-3. Toutefois, on préfère les désigner comme étant des dérivés N-(vinblastinoyl-23) d'acide amino-1 méthyl phosphonique.

Parmi les acides pharmaceutiquement acceptables pour la préparation des sels d'addition aux composés de formule générale I, on peut citer les acides phosphorique, chlorhydrique, citrique, oxalique, maléïque, sulfurique, tartrique, mandélique, fumarique, méthanesulfonique etc...

Les composés selon l'invention, ainsi que leurs sels d'addition, sont doués de propriétés pharmacologiques fort intéressantes, et se distinguent des autres dérivés N-(vinblastinoyl-23) d'acides aminés déjà connus.

Les composés de l'invention ont été testés par leur capacité à prolonger la survie de souris porteuses de cellules tumorales (P 388 et L 1210) par voie intrapéritionéale selon les protocoles recommandés par l'Institut National du Cancer USA (Geran R.I. et coll., Cancer Chemotherapy Reports,(1972),III,3,N°2,p.1-87), et reconnus comme représentatifs de l'effet antitumoral en clinique humaine (Driscoll J.S. Cancer Treatment Reports,(1984),68,N°1,p.63-85 et "In vivo cancer Models" US Department of Health and Human Services NIH Publication N° 84-2635 Feb. 1984).

Les composés de la présente invention se sont révélés non seulement capables de ralentir la croissance des tumeurs greffées chez la souris, mais également de guérir des animaux leucémiques. En effet, de nombreuses rémissions complètes ont été observées. En plus, des essais comparatifs avec des produits de références décrits dans la littérature-vinblastine et vintryptol- ont démontré que les composés de l'invention ont une activité très supérieure par rapport aux composés déjà connus.

Les composés de la présente invention sont utiles chez l'homme et les animaux en cas de maladie de Hodgkin, lymphomes non hodgkiniens, cancers du testicule, épithélioma du sein et de l'ovaire, sarcome de Kaposi, choriocarcinome histocytose, rhabdomyosarcomes, neuroblastomes, tumeurs de Wilms, sarcomes d'Ewing etc... D'autres applications thérapeutiques peuvent être aussi envisagées pour les composés de l'invention. En effet, il est connu que les alcaloïdes bisindoliques et leurs dérivés sont actifs pour le traitement de certaines arthrites ou de psoriasis. (Brevets US 4,208,411 et 3,749,784).

L'invention s'étend aussi aux compositions pharmaceutiques renfermant comme principe actif au moins un composé de formule générale I, l'un de ses isomères optiques ou l'un de ses sels d'addition avec un acide

minéral ou organique avec un ou plusieurs excipients, inertes, non toxiques et appropriés.

Les compositions pharmaceutiques ainsi obtenues sont présentées avantageusement sous des formes diverses telles que par exemple comprimés, dragées, gélules, crèmes pour applications locales, suppositoires, solutions injectables etc... Elles peuvent contenir des doses de 0,1 à 100 mg d'un ou plusieurs composés de l'invention.

Pour leur application thérapeutique, les composés de l'invention, leurs isomères optiques ou leurs sels d'addition, sont de préférence administrés par voie parentérale. D'une manière générale, les composés de l'invention peuvent être utilisés en s'inspirant des techniques et des limitations qui sont connues pour les traitements thérapeutiques avec les autres alcaloïdes de la classe des Vinca.

La posologie peut varier largement en fonction de l'âge, du poids du patient, de la nature et de la sévérité de l'affection, de la voie d'administration ainsi que du schéma thérapeutique utilisé. Les doses hebdomadaires totales s'échelonneront généralement de 0,05 à environ 20 mg/kg.

Les composés de l'invention peuvent être utilisés seuls ou en combinaison avec un ou plusieurs agents carcinostatiques incluant par exemple les agents alkylants, les antimétabolites tels que le méthotrexate, le fluoro-5-uracile, la mercapto-6 purine, la thio-6 guanine, les arabinosides de la cytosine et les antibiotiques tels que l'actinomycine D, la daunorubicine, l'adriamycine, et le cis-diammine dichloro platine, etc...

Les exemples suivants, donnés à titre non-limitatif, illustrent l'invention.

Les spectres de résonance magnétique nucléaire (R.M.N.) du proton ont été enregistrés à 400 MHz.

Les points de fusion indiqués sont mesurés selon la technique micro-Köfler.

Les spectres de masse (F.A.B.$^+$ et F.A.B.$^-$) sont obtenus avec un spectromètre de masse à filtre quadripolaire, NERMAG ® 10-10C.

Matrice : mélange glycérol et thioglycérol (50V/50V).
Gaz incident : Krypton
Energie : 6 à 8 keV.

Les principaux fragments des spectres de masses des composés des exemples 1-11 décrits ci-dessous sont indiqués dans le Tableau I.

**EXEMPLE 1**

**N-(désacétyl-0-4 vinblastinoyl-23) amino-1 méthyl-2 propyl phosphonate de diéthyle**

A une solution de 130 ml d'acide chlorhydrique N, refroidie à 0°C et contenant 2,34 mmoles de désacétyl-0-4 vinblastinoyl-23 hydrazide, on ajoute 5,20 mmoles de nitrite de sodium. Après dix minutes de contact à 0°C, on ajuste le pH du milieu à 8,8 à l'aide d'une solution glacée et saturée de bicarbonate de sodium et on extrait rapidement à l'aide de 4 fois 100 ml de dichlorométhane. Les phases organiques réunies sont lavées à l'aide d'une solution saturée de chlorure de sodium et séchées sur sulfate de magnésium anhydre. On concentre la phase organique jusqu'à un volume de 50 ml, on ajoute 3,10 mmoles d'amino-1 méthyl-2 propyl phosphonate de diéthyle (Synthesis,(1981),57) et on abandonne le milieu réactionnel 24 heures à température ambiante.

Après évaporation du solvant, le résidu est purifié par chromatographie sur colonne de silice (230-400 Mesh) en utilisant comme éluant un mélange de toluène et d'éthanol (80V/20V).

On recueille 0,67 g de produit que l'on cristallise dans un mélange d'éther éthylique et d'éther de pétrole (50V/50V).

Rendement : 30%

**Sulfate de (+) N-(désacétyl-0-4 vinblastinoyl-23) amino-1 méthyl-2 propyl phosphonate de diéthyle**

Ce composé est préparé selon la méthode décrite ci-dessus à partir de 1,5 g de N-(désacétyl-0-4-vinblastinoyl-23) hydrazide et de 0,65 g de (+) amino-1 méthyl-2 propyl phosphonate de diéthyle.

Après 24 heures d'agitation à température ambiante, le solvant est séparé pour obtenir 1,6 g de produit que l'on dissout dans 4 ml d'éthanol. Cette solution est ensuite purifiée par chromatographie en utilisant une colonne contenant 500 g de Lichroprep RP 18 (15-25 μm). On élue à l'aide d'un mélange de méthanol et de solution aqueuse d'hydrogenophosphate disodique 0,1M (70V/30V). Le débit de la phase mobile est fixé à 20 ml/min. Les fractions 620 à 800 sont rassemblées et après condensation sous vide, le résidu est extrait par le chlorure de méthylène, puis, la phase organique est séchée sur sulfate de magnésium anhydre. Après évaporation du solvant, on obtient 0,55 g de (+) N-(désacétyl-0-4-vinblastinoyl-23) amino-1 méthyl-2 propyl phosphonate de diéthyle que l'on salifie ensuite à l'aide de l'éthanol sulfurique à 2%.

Rendement : 30%

Spectre de résonance magnétique nucléaire du proton (base - solvant $CDCl_3$)

| | | | |
|---|---|---|---|
| 0,89 ppm,6H; | 1,81 ppm,1H; | 3,21 ppm,1H; | 4,29 ppm,1H; |
| 1,08 ppm,6H; | 2,10 ppm,2H; | 3,34 ppm,1H; | 4,33 ppm,1H; |
| 1,20 ppm,1H; | 2,30 ppm,2H; | 3,42 ppm,1H; | 5,68 ppm,1H; |
| 1,31 ppm,6H; | 2,38 ppm,1H; | 3,45 ppm,1H; | 5,81 ppm,1H; |
| 1,35 ppm,2H; | 2,53 ppm,1H; | 3,59 ppm,3H; | 6,09 ppm,1H; |
| 1,42 ppm,1H; | 2,79 ppm,3H; | 3,76 ppm,3H; | 6,57 ppm,1H; |
| 1,50 ppm,1H; | 2,80 ppm,2H; | 3,95 ppm,1H; | 7,13 ppm,3H; |
| 1,64 ppm,1H; | 2,83 ppm,1H; | 4,13 ppm,2H; | 7,47 ppm,1H; |
| 1,80 ppm,1H; | 3,13 ppm,1H; | 4,18 ppm,1H; | 7,51 ppm,1H. |

**Sulfate de (-) N-(désacétyl-0-4 vinblastinoyl-23) amino-1 méthyl-2 propyl phosphonate de diéthyle**

Ce composé est obtenu selon le procédé décrit pour le sulfate de (+) N-(désacétyl-0-4 vinblastinoyl-23) amino-1 méthyl-2 propyl phosphonate de diéthyle mais en utilisant le (-) amino-1 méthyl-2 propyl phosphonate de diéthyle.

Lors de la purification par chromatographie, les fractions 400 à 500 sont réunies, et traitées comme décrit ci-dessus.

Rendement : 29%

Spectre de résonnance magnétique nucléaire du proton (base - solvant $CDCl_3$)

| | | | |
|---|---|---|---|
| 0,89 ppm,3H; | 2,05 ppm,1H; | 3,24 ppm,1H; | 4,41 ppm,1H; |
| 0,93 ppm,3H; | 2,18 ppm,1H; | 3,35 ppm,1H; | 5,74 ppm,1H; |
| 1,00 ppm,6H; | 2,28 ppm,2H; | 3,45 ppm,1H; | 5,84 pmm,1H; |
| 1,25 ppm,1H; | 2,41 ppm,1H; | 3,59 pmm,3H; | 6,07 pmm,1H; |
| 1,34 pmm,8H; | 2,56 ppm,1H; | 3,77 ppm,3H; | 6,58 ppm,1H; |
| 1,45 ppm,1H; | 2,81 ppm,1H; | 3,96 ppm,1H; | 7,10 à 7,13 ppm,3H; |
| 1,50 ppm,1H; | 2,83 ppm,5H; | 4,16 ppm,2H; | 7,40 ppm,1H; |
| 1,67 ppm,1H; | 3,13 ppm,2H; | 4,20 ppm,2H; | 7,52 ppm,1H. |
| 1,79 ppm,1H | | | |

**EXEMPLES 2-11**

Les composés des exemples 2 à 11 sous forme de mélange de diastéréo-isomères ont été préparés selon le procédé décrit dans l'exemple 1 en utilisant les dérivés d'amino-1 méthyl phosphonate d'alkyle adéquats.

**EXEMPLE 2**

**N-(désacétyl-0-4 vinblastinoyl-23) amino-1 éthyl phosphonate de diéthyle**

Dérivé d'amino-1 méthyl phosphonate d'alkyle utilisé : amino-1 éthyl phosphonate de diéthyle

Rendement : 30%

**Sulfate du (-) N-(désacétyl-0-4 vinblastinoyl-23) amino-1 éthyl phosphonate de diéthyle**

Ce composé est obtenu selon le procédé décrit pour le sulfate de (-) N-(désacétyl-0-4 vinblastinoyl-23) amino-1 méthyl-2 propyl phosphonate de diéthyle et en utilisant le (-) amino-1 éthyl phosphonate de diéthyle. Lors de la purification par chromatographie les fractions retenues sont : 164 à 209.

Spectre de résonance magnétique nucléaire du proton (base - solvant $CDCl_3$)

| | | | |
|---|---|---|---|
| 0,896 ppm,3H; | 2,29 ppm,1H; | 3,33 ppm,1H; | 4,47 ppm,1H; |
| 0,94 ppm,3H; | 2,44 ppm,1H; | 3,43 ppm,1H; | 5,79 ppm,1H; |
| 1,25 ppm,1H; | 2,59 ppm,1H; | 3,60 ppm,3H; | 5,84 ppm,1H; |
| 1,34 ppm,6H; | 2,84 ppm,3H; | 3,75 ppm,1H; | 6,07 ppm,1H; |
| 1,38 ppm,3H; | 2,87 ppm,1H; | 3,77 ppm,3H; | 6,57 ppm,1H; |
| 1,73 ppm,2H; | 3,14 ppm,1H; | 3,95 ppm,1H; | 7,10 à 7,15 ppm,3H; |
| 2,03 ppm,1H; | 3,23 ppm,1H; | 4,17 ppm,2H; | 7,52 ppm,1H. |

Spectre de masse:

(matrice mélange glycérol et thioglycérol 50V/50V - atomes bombardants : xenon).
$FAB^+$ (m/z) : 932 (M+14+1); 918 (M+1); 900; 886; 858; 709; 542; 355; 337; 323; 295; 154; 144, 122; 108
$FAB^-$ (m/z) : 930 (M+14+1); 916 (M-1); 914; 898; 888; 886; 870; 858; 778; 760; 178; 152; 137.

**Sulfate du (+) N-(désacétyl-0-4 vinblastinoyl-23) amino-1 éthyl phosphonate de diéthyle**

Ce composé est obtenu selon le procédé décrit pour le sulfate de (+) N-(désacétyl-0-4 vinblastinoyl-23) amino-1 méthyl-2 propyl phosphonate de diéthyle et en utilisant le (+) amino-1 éthyl phosphonate de diéthyle. Lors de la purification, les fractions recueillies sont : 221 à 256.

Spectre de résonance magnétique nucléaire du proton (base - solvant $CDCl_3$)

| | | | |
|---|---|---|---|
| 0,89 ppm,3H; | 2,03 ppm,1H; | 3,21 ppm,1H; | 4,20 ppm,1H; |
| 0,92 ppm,3H; | 2,29 ppm,1H; | 3,33 ppm,1H; | 4,50 ppm,1H; |
| 1,23 ppm,1H; | 2,42 ppm,1H; | 3,39 ppm,1H; | 5,72 ppm,1H; |
| 1,30 ppm,1H; | 2,57 ppm,1H; | 3,59 ppm,3H; | 5,82 ppm,1H; |
| 1,32 ppm,6H; | 2,78 ppm,3H; | 3,77 ppm,3H; | 6,08 ppm,1H; |
| 1,40 ppm,2H; | 2,80 ppm,1H; | 3,78 ppm,1H; | 6,57 ppm,1H; |
| 1,43 ppm,3H; | 2,83 ppm,1H; | 3,95 ppm,1H; | 7,10 à 7,15 ppm,3H; |
| 1,68 ppm,1H; | 3,14 ppm,1H; | 4,14 ppm,2H; | 7,51 ppm,1H |
| 1,77 ppm,1H. | | | |

Spectre de masse

(matrice mélange glycérol et thioglycérol 50V/50V - atomes bombardants : xenon)
$FAB^+$ (m/z) : 932 (M+14+1); 918 (M+1); 900; 886; 858; 651; 649; 571; 355; 337; 323; 295; 154; 144; 122; 108.
$FAB^-$ (m/z) : 930 (M+14+1); 916 (M-1); 914; 888; 886; 870; 858; 840; 778; 760; 178; 152; 137.

**EXEMPLE 3**

**N-(désacétyl-0-4 vinblastinoyl-23) amino-1 hexyl phosphonate de diéthyle**

Dérivé d'amino-1 méthyl phosphonate d'alkyle utilisé : amino-1 hexyl phosphonate de diéthyle

Rendement : 35%

Pour former le sulfate correspondant, le N-(désacétyl-0-4 vinblastinoyl-23) amino-1 hexyl phosphonate de diéthyle est salifié à l'aide d'éthanol sulfurique à 2%. Le sel formé est précipité par addition d'éther éthylique.

Point de fusion : 200°C.

**EXEMPLE 4**

**N-(désacétyl-0-4 vinblastinoyl-23) amino-1 méthyl-3 butyl phosphonate de diéthyle**

Dérivé d'amino-1 méthyl phosphonate d'alkyle utilisé : amino-1 méthyl-3 butyl phosphonate de diéthyle

Rendement : 31%

Pour former le sulfate correspondant, ce composé est ensuite dissous dans l'alcool sulfurique à 2% et le sel formé est précipité à l'aide d'éther éthylique.

Point de fusion : 202-206°C (décomposition).

**EXEMPLE 5**

**N-(désacétyl-0-4 vinblastinoyl-23) amino-1 butène-3 yl phosphonate de diéthyle**

Dérivé d'amino-1 méthyl phosphonate d'alkyle utilisé : amino-1 butène-3 yl phosphonate de diéthyle

Rendement : 25%

Le sulfate de N-(désacétyl-0-4 vinblastinoyl-23) amino-1 butène-3 yl phosphonate de diéthyle a été préparé selon le procédé de salification décrit ci-dessus.

Point de fusion : 196-200°C.

**Séparation du mélange de diastéréo-isomères de N-(désacétyl-0-4 vinblastinoyl-23) amino-1 butène-3-yl phosphonate de diéthyle**

Une solution de N-(désacétyl-0-4 vinblastionyl-23) amino-1 butène-3-yl phosphonate de diéthyle à 20% dans l'éthanol contenant 600 mg de produit, est chromatographiée sur colonne contenant 500 g de Lichroprep RP18 15-25 μm, en utilisant comme solvant d'élution un mélange de méthanol et d'une solution aqueuse d'hydrogénophosphate disodique 0,1M (65V/35V). Le débit de la phase mobile est fixé à 20 ml/min et le volume des fractions recueillies est de 20 ml.

**Diastéréo-isomère A**

Les fractions 90 à 130 sont rassemblées, on évapore le méthanol sous vide, on extrait au chlorure de méthylène, on sèche la phase organique sur sulfate de magnésium anhydre et on évapore le solvant pour obtenir le diastéréo-isomère A.

Spectre de résonance magnétique nucléaire du proton (base - solvant $CDCl_3$)

| | | | | | | | |
|---|---|---|---|---|---|---|---|
| 0,89 | ppm,3H; | 2,41/2,62 | ppm,2H; | 3,59 | ppm,3H; | 6,07 | ppm,1H; |
| 0,90 | ppm,1H; | 2,41/3,22 | ppm,2H; | 3,77 | ppm,3H; | 6,57 | ppm,1H; |
| 0,93 | ppm,3H; | 2,41/3,41 | ppm,2H; | 4,15 | ppm,2H; | 7,14 | ppm,2H; |
| 1,25/1,68 | ppm,2H; | 2,58 | ppm,1H; | 4,18 | ppm,1H; | 7,34 | pmm,1H; |
| 1,30 | ppm,2H; | 2,82 | ppm,5H; | 4,52 | ppm,1H; | 7,52 | ppm,1H; |
| 1,34 | ppm,6H; | 2,85/3,35 | ppm,2H; | 5,1 | ppm,2H; | 8,03 | ppm,1H; |
| 1,41/1,49 | ppm,2H; | 3,12/3,30 | ppm,2H; | 5,74 | ppm,1H; | 9,55 | ppm,1H. |
| 1,70/2,03 | ppm,2H; | 3,12/3,68 | ppm,2H; | 5,81 | ppm,1H; | | |
| 2,27/3,96 | ppm,2H; | 3,44 | ppm,1H; | 5,83 | ppm,1H; | | |

**Diastéréo-isomère B**

On traite de la même manière les fractions 170 à 220 pour obtenir le diastéréo-isomère B.

Spectre de résonance magnétique nucléaire du proton (base - solvant $CDCl_3$) :

| | | | | | |
|---|---|---|---|---|---|
| 0,87 | ppm,3H; | 2,53 | ppm,1H; | 4,49 | ppm,1H; |
| 0,89 | ppm,3H; | 2,75 | ppm,3H; | 5,10 | ppm,2H; |
| 0,90 | ppm,1H | 2,8/3,3 | ppm,2H; | 5,68 | ppm,1H; |
| 1,22/1,64 | ppm,2H; | 2,80 | ppm,2H; | 5,80 | ppm,2H; |
| 1,30 | ppm,6H; | 3,11/3,39 | ppm,2H; | 6,07 | ppm,1H; |
| 1,35 | ppm,2H; | 3,20/3,72 | ppm,2H; | 6,57 | ppm,1H; |
| 1,48 | ppm,2H; | 3,34 | ppm,1H; | 7,10 | ppm,2H; |
| 1,78/2,02 | ppm,2H; | 3,57 | ppm,3H; | 7,32 | ppm,1H; |
| 2,40/2,70 | ppm,2H; | 3,75 | ppm,3H; | 7,50 | ppm,1H; |
| 2,40/3,95 | ppm,2H; | 4,14 | ppm,2H; | 8,02 | ppm,1H; |
| 2,40/3,39 | ppm,2H; | 4,20 | ppm,1H; | 9,57 | ppm,1H. |

## EXEMPLE 6

**N-(désacétyl-0-4 vinblastinoyl-23) amino-1 phényl-2 éthyl phosphonate de diéthyle**

Dérivé d'amino-1 méthyl phosphonate d'alkyle utilisé : amino-1 phényl-2 éthyl phosphonate de diéthyle

Rendement : 40%

Pour former le sulfate de N-(désacétyl-0-4 vinblastinoyl-23 amino-1 phényl-2 éthyl phosphonate de diéthyle, l'amine a été salifiée à l'aide de l'alcool sulfurique à 2% selon le procédé décrit précédemment.

Point de fusion : 199°C.

**Séparation du mélange de diastéréo-isomères de N-(désacétyl-0-4 vinblastinoyl-23) amino-1 phényl-2 éthyl phosphonate de diéthyle**

Les diastéréo-isomères de N-(désacétyl-0-4 vinblastinoyl-23) amino-1 phényl-2 éthyl phosphonate de diéthyle ont été obtenus selon la méthode décrite dans l'example 5.
Solvant d'élution : mélange de méthanol et d'une solution aqueuse d'hydrogénophosphate disodique 0,1M (70V/30V).

**Diastéréo-isomère A**

Ce composeé est obtenu par les fractions 120 à 165.

Spectre de résonance magnétique nucléaire du proton (base - solvant $CDCl_3$)

| | | | | | | | |
|---|---|---|---|---|---|---|---|
| 0,87 | ppm,1H; | 2,4/3,22 | ppm,2H; | 3,42 | ppm,1H; | 6,04 | ppm,1H; |
| 0,9 | ppm,6H; | 2,4/3,4 | ppm,2H; | 3,58 | ppm,3H; | 6,57 | ppm,1H; |
| 1,21/1,66 | ppm,2H; | 2,57 | ppm,1H; | 3,75 | ppm,3H; | 7,10 | ppm,1H |
| 1,31 | ppm,2H; | 2,78 | ppm,3H; | 4,01 | ppm,1H; | 7,15 | ppm,2H, |
| 1,32 | ppm,3H; | 2,83/3,34 | ppm,2H; | 4,12 | ppm,2H; | 7,20 | ppm,1H; |
| 1,38/1,5 | ppm,2H; | 2,83 | ppm,2H; | 4,70 | ppm,1H; | 7,28 | ppm,2H; |
| 1,74/2,02 | ppm,2H; | 2,90/3,21 | ppm,2H; | 5,73 | ppm,1H; | 7,30 | ppm,2H; |
| 2,26/3,95 | ppm,2H; | 3,15/3,29 | ppm,2H; | 5,83 | ppm,1H; | 7,53 | ppm,1H; |

**Diastéréo-isomère B**

Ce composé est obtenu par les fractions 245 à 300.

Spectre de résonance magnétique nucléaire du proton (base - solvant $CDCl_3$)

| | | | | | |
|---|---|---|---|---|---|
| 0,84 | ppm,1H; | 2,80/3,30 | ppm,2H; | 4,68 | ppm,1H; |
| 0,88 | ppm,3H; | 2,80/3,40 | ppm,2H; | 5,67 | ppm,1H; |
| 0,89 | pmm,3H; | 2,81 | ppm,2H; | 5,80 | ppm,1H; |
| 1,15/1,60 | ppm,2H; | 3,11/3,30 | ppm,2H; | 5,91 | ppm,1H; |
| 1,32 | ppm,5H; | 3,11/3,65 | ppm,2H; | 6,52 | ppm,1H; |
| 1,45 | ppm,2H; | 3,46 | ppm,1H; | 7,11 | ppm,1H; |
| 1,72/1,91 | ppm,2H; | 3,56 | ppm,3H; | 7,16 | ppm,2H; |
| 2,23/3,94 | ppm,2H; | 3,71 | ppm,3H; | 7,25 | ppm,2H; |
| 2,39/3,21 | ppm,2H; | 4,04 | ppm,1H; | 7,34 | ppm,1H; |
| 2,39/3,39 | ppm,2H; | 4,17 | ppm,2H; | 7,51 | ppm,1H; |
| 2,51 | ppm,1H; | | | | |

**EXEMPLE 7**

**N-(désacétyl-0-4 vinblastinoyl-23) amino-1 (indolyl-3)-2 éthyl phosphonate de diméthyle**

Dérivé d'amino-1 méthyl phosphonate d'alkyle utilisé : amino-1 (indolyl-3)-2 éthyl phosphonate de diméthyle. Ce dernier composé a été synthétisé selon la méthode décrite dans Tetrahedron Letters, (1983),24, p.5461.

Rendement : 47%

**EXEMPLE 8**

**(+) N-(désacétyl-0-4 vinblastinoyl-23) amino-1 (indolyl-3)-2 éthyl phosphonate de diéthyle**

STADE A

(+) Amino-1 (indolyl-3)-2 éthyl phosphonate de diéthyle

0,024 moles d'amino-1 (indolyl-3)-2 éthyl phosphonate de diéthyle, préparé selon le procédé décrit dans Tetrahedron Letters, (1983),24, p.5461, sont diluées dans 25 ml d'éthanol absolu. On ajoute 50 ml d'éthanol absolu contenant 0,024 moles de monohydrate de l'acide (-) dibenzoyltartrique. Après agitation du milieu réactionnel pendant une heure, le précipité formé est filtré, lavé à l'éthanol, et séché sous vide. Après recristallisation dans l'éthanol absolu, on obtient un sel dont le point de fusion est 217-218°C.

Ce sel est mis en suspension dans du chlorure de méthylène. On ajoute ensuite, goutte à goutte, une solution d'ammoniaque à 25% jusqu'à l'obtention d'un pH égal à 9.

La phase organique est ensuite décantée et séchée sur sulfate de magnésium anhydre. Après évaporation du solvant, on obtient 1,6 g du produit attendu, sous forme d'huile.

Rendement : 80%.

Pouvoir rotatoire : $\alpha_D^{22}$ = + 18,6° (solution à 1% dans $CHCl_3$)

STADE B

Le (+) N-(désacétyl-0-4 vinblastinoyl-23) amino-1 (indolyl-3)-2 éthyl phosphonate de diéthyle est obtenu, selon le procédé décrit dans l'exemple 1, en utilisant 1,95 mmoles de désacétyl-0-4 vinblastinoyl-23 hydrazide, et 2 mmoles du composé obtenu au stade précédent.

Rendement : 55%

Spectre de résonance magnétique nucléaire du proton (base - solvant $CDCl_3$) :

| | | | | |
|---|---|---|---|---|
| 0,88 ppm,6H; | 1,60 ppm,2H; | 2,63 ppm,1H; | 3,46 ppm,1H; | 5,78 ppm,1H; |
| 0,92 ppm,1H; | 1,64 ppm,3H; | 2,70 ppm,1H; | 3,55 ppm,3H; | 5,83 ppm,1H; |
| 1,13 ppm,1H; | 1,72 ppm,1H; | 2,83 ppm,1H; | 3,64 ppm,3H; | 6,46 ppm,1H; |
| 1,31 ppm,2H; | 2,24 ppm,1H; | 3,14 ppm,1H; | 3,95 ppm,2H; | 7,12 ppm,1H; |
| 1,32 ppm,2H; | 2,38 ppm,1H; | 3,20 ppm,1H; | 4,18 ppm,4H; | 7,47 ppm,1H; |
| 1,34 ppm,2H; | 2,40 ppm,1H; | 3,30 ppm,2H; | 4,67 ppm,1H; | 7,97 ppm,1H; |
| 1,35 ppm,1H; | 2,47 ppm,1H; | 3,45 ppm,1H; | 5,67 ppm,1H; | 9,49 ppm,1H; |
| 1,47 ppm,1H. | | | | |

Le sulfate de (+) N-(désacétyl-0-4 vinblastinoyl-23) amino-1 (indolyl-3)-2 éthyl phosphonate de diéthyle a été préparé selon la méthode décrite dans J.Med.Chem.,(1978),21,p.96, après addition de 4,35 ml d'éthanol

sulfurique à 2%. Le sel est précipité à l'aide d'éther éthylique.
Point de fusion : 220-228°C (décomposition)

**EXEMPLE 9**

**(-) N-(désacétyl-0-4 vinblastinoyl-23) amino-1 (indolyl-3)-2 éthyl phosphonate de diéthyle**

STADE A

(-) amino-1 (indolyl-3)-2 éthyl phosphonate de diéthyle

Ce composé a été préparé selon le procédé décrit dans l'exemple 10, Stade A, mais en utilisant l'acide (+) dibenzoyltartrique au lieu de son isomère lévogyre.
Rendement : 82%
Pouvoir rotatoire : $\alpha_D^{22}$ = -18° (solution à 1% dans $CHCl_3$)

STADE B

Le (-) N-(désacétyl-0-4 vinblastinoyl-23) amino-1 (indolyl-3)-2 éthyl phosphonate de diéthyle a été préparé à partir de 1,95 mmoles de désacétyl-0-4 vinblastinoyl hydrazide et de 2 mmoles du composé obtenu au stade précédent.
Rendement : 54%

Spectre de résonance magnétique nucléaire du proton (base - solvant $CDCl_3$) :

| | | | | |
|---|---|---|---|---|
| 0,81 ppm,3H; | 1,51 ppm,1H; | 3,28 ppm,1H; | 4,17 ppm,4H; | 7,15 ppm,1H; |
| 0,85 ppm,3H; | 2,81 ppm,3H; | 3,37 ppm,1H; | 4,79 ppm,1H; | 7,50 ppm,1H; |
| 1,15 ppm,2H; | 2,83 ppm,1H; | 3,42 ppm,1H; | 5,60 ppm,1H; | 7,99 ppm,1H; |
| 1,33 ppm,6H; | 2,86 ppm,2H; | 3,56 ppm,3H; | 5,80 ppm,1H; | 8,50 ppm,1H; |
| 1,37 ppm,1H; | 3,08 ppm,1H; | 3,73 ppm,3H; | 6,01 ppm,1H; | 9,50 ppm,1H. |

Le sulfate de (-) N-(désacétyl-0-4 vinblastinoyl-23) amino-1 (indolyl-3)-2 éthyl phosphonate de diéthyle a été obtenu après addition d'une quantité adéquate d'éthanol sulfurique à 2%.
Point de fusion : 198-202°C (décomposition)

**EXEMPLE 10**

**N-(désacétyl-0-4 vinblastinoyl-23) aminométhyl phosphonate de diéthyle**

Dérivé d'amino-1 méthyl phosphonate d'alkyle utilisé : aminométhyl phosphonate de diéthyle
Rendement : 30%
Le sulfate de N-(désacétyl-0-4 vinblastinoyl-23) aminométhyl phosphonate de diéthyle a été obtenu après addition d'éthanol sulfurique à 2%.
Point de fusion : 194-204°C (décomposition).

**EXEMPLE 11**

**N-(désacétyl-0-4 vinblastinoyl-23) amino-1 carboéthoxy-2 éthyl phosphonate de diéthyle**

Dérivé d'amino-1 méthyl phosphonate d'alkyle utilisé : amino-1 carboéthoxy-2 éthyl phosphonate de diéthyle.

Rendement : 47%

Le sulfate correspondant a été obtenu par addition d'éthanol sulfurique à 2%, comme indiqué ci-dessus.

Point de fusion : 208-210°C (décomposition).

## EXEMPLE 12

### N-(désacétyl-0-4 vinblastinoyl-23) amino-1 éthyl phosphonate de diéthyle $N_b$'-oxyde

**(-) N-(désacétyl-0-4) vinblastinoyl-23) amino-1 éthyl phosphonate de diéthyle $N_b$'-oxyde**

Le (-) N-(désacétyl-0-4 vinblastinoyl-23) amino-1 éthyl phosphonate de diéthyle, composé décrit dans l'exemple 2 est repris dans un mélange d'éther éthylique et d'éther de pétrole (50:50 V/V) saturé d'oxygène. Le produit cristallin ainsi obtenu est ensuite filtré et séché.

Rendement : 100%

Spectre de résonance magnétique nucléaire du proton (base - solvant $CDCl_3$) :

| | | | | |
|---|---|---|---|---|
| 0,76 ppm,3H; | 1,63 ppm,1H; | 2,85 ppm,3H; | 3,80 ppm,2H; | 4,25 ppm,1H; |
| 0,97 ppm,3H; | 1,70 ppm,2H; | 3,25 ppm,1H; | 4,00 ppm,1H; | 4,46 ppm,1H; |
| 1,10 ppm,1H; | 2,05 ppm,1H; | 3,30 ppm,1H; | 4,04 ppm,1H; | 5,72 ppm,1H; |
| 1,35 ppm,3H; | 2,41 ppm,1H; | 3,45 ppm,1H; | 4,10 ppm,1H; | 5,81 ppm,1H; |
| 1,39 ppm,3H; | 2,50 ppm,1H; | 3,67 ppm,3H; | 4,11 ppm,1H; | 6,06 ppm,1H; |
| 1,50 ppm,1H; | 2,60 ppm,1H; | 3,79 ppm,3H; | 4,17 ppm,2H; | 6,17 ppm,1H; |
| 1,60 ppm,1H; | 2,74 ppm,1H. | | | |

**(+) N-(désacétyl-0-4 vinblastinoyl-23) amino-1 éthyl phosphonate de diéthyle $N_b$'-oxyde**

Le (+) N-(désacétyl-0-4 vinblastinoyl-23) amino-1 éthyl phosphonate de diéthyle est traité selon le procédé décrit ci-dessus pour obtenir le N-(désacétyl-0-4 vinblastinoyl-23) amino-1 éthyl phosphonate de diéthyle $N_b$'-oxyde.

Rendement : 100%

Spectre de résonance magnétique nucléaire du proton (base - solvant $CDCl_3$) :

| | | | | |
|---|---|---|---|---|
| 0,74 ppm,3H; | 1,58 ppm,1H; | 2,70 ppm,1H; | 3,77 ppm,3H; | 4,49 ppm,1H; |
| 0,93 ppm,3H; | 1,60 ppm,2H; | 2,78 ppm,3H; | 3,78 ppm,1H; | 5,65 ppm,1H; |
| 1,05 ppm,1H; | 1,70 ppm,1H; | 3,20 ppm,2H; | 3,80 ppm,1H; | 5,77 ppm,1H; |
| 1,08 ppm,1H; | 1,80 ppm,1H; | 3,40 ppm,1H; | 4,00 ppm,2H; | 6,06 ppm,1H; |
| 1,30 ppm,3H; | 2,03 ppm,1H; | 3,60 ppm,1H; | 4,10 ppm,2H; | 6,16 ppm,1H; |
| 1,42 ppm,3H; | 2,40 ppm,2H; | 3,65 ppm,3H; | 4,15 ppm,2H; | |
| 1,50 ppm,1H; | 2,56 ppm,1H; | 3,70 ppm,1H; | 4,23 ppm,1H. | |

TABLEAU I

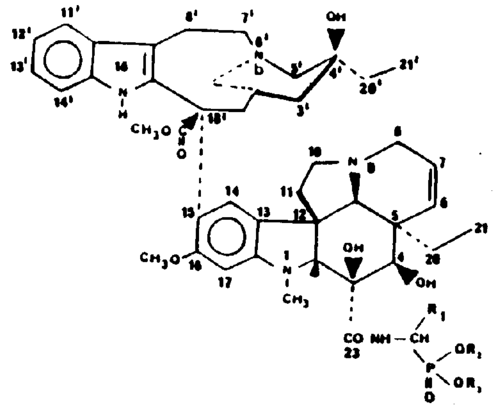

| COMPOSE | $R_1$ | $R_2$ | $R_3$ | SPECTRE DE MASSE (m/z) |
| --- | --- | --- | --- | --- |
| Exemple 1 (Mélange de dias-téréo-isomères) | 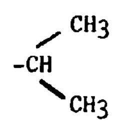 | $-C_2H_5$ | $-C_2H_5$ | FAB $^+$ : 946 (M+1), 709, 651<br>FAB $^-$ : 944 (M-1),926,916,806,180,152, 137,108 |
| Exemple 2 (Mélange de dias-téréo-isomères) | $-CH_3$ | $-C_2H_5$ | $-C_2H_5$ | FAB + : 932,918 (M+1),900,886,872,858<br>FAB - : 930,916 (M-1),898,888,858,137 |

TABLEAU I (SUITE 1)

| COMPOSE | $R_1$ | $R_2$ | $R_3$ | SPECTRE DE MASSE (m/z) |
|---|---|---|---|---|
| Exemple 3 | $-C_5H_{11}$ | $-C_2H_5$ | $-C_2H_5$ | FAB + : 974 (M+1),914,836,651,649,632,619,571,542,475,238,189,176,104<br>FAB - : 972 (M-1),944,914,834,321,208,196,190,180,154,137 |
| Exemple 4 | 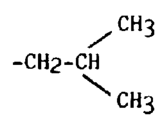 | $-C_2H_5$ | $-C_2H_5$ | FAB + : 974,960 (M+1),944,928,900,709,651,649,604,571,542,355<br>FAB - : 958 (M-1),930,820,137 |
| Exemple 5 (Mélange de dias-téréo-isomères) | $-CH_2-CH=CH_2$ | $-C_2H_5$ | $-C_2H_5$ | FAB + : 944 (M+1),806,709,651,602,590,571,542,355,295,272<br>FAB - : 942 (M-1),137 |
| Exemple 6 (Mélange de dias-téréo-isomères) | 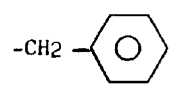 | $-C_2H_5$ | $-C_2H_5$ | FAB + : 994 (M+1),962,934,856<br>FAB - : 992 (M-1),964,854,137 |

TABLEAU I (SUITE 2)

| COMPOSE | $R_1$ | $R_2$ | $R_3$ | SPECTRE DE MASSE (m/z) |
|---|---|---|---|---|
| Exemple 7 | $-CH_2$ 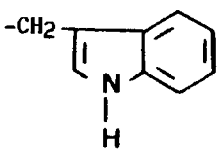 | $-CH_3$ | $-CH_3$ | FAB + : 1019,1005 (M+1),754,752,651,355 |
| Exemple 8 | $-CH_2$ 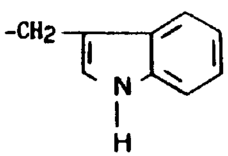 | $-C_2H_5$ | $-C_2H_5$ | FAB + : 1033 (M+1),709,679,651,571,542, 517,355,297 |
| Exemple 9 | $-CH_2$ 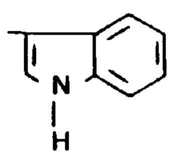 | $-C_2H_5$ | $-C_2H_5$ | FAB + : 1047,1033 (M+1),709,691,679, 651,571,542,355,295,272,130,124<br>FAB − : 1031 (M-1),1003 |

TABLEAU I (SUITE 3)

| COMPOSE | $R_1$ | $R_2$ | $R_3$ | SPECTRE DE MASSE (m/z) |
|---|---|---|---|---|
| Exemple 10 | H | $-C_2H_5$ | $-C_2H_5$ | FAB + : 904 (M+1),651,562,550,355 |
| Exemple 11 | $-CH_2COOC_2H_5$ | $-C_2H_5$ | $-C_2H_5$ | FAB + : 990 (M+1),752,651,649,571,542,355<br>FAB - : 988 (M-1),960,752,662 |

## ETUDE PHARMACOLOGIQUE

### EXEMPLE 13

### Activité antitumorale sur la leucémie P 388 chez la souris

Deux groupes de souris (n=6), de souche $B_6D_2F_1$ ($F_1$:C57$Bl_6$ X $DBA_2$), de même sexe et âge, ont reçu, par voie intrapéritonéale, au jour zéro, 0,4ml de sérum physiologique contenant en suspension $10^6$ de cellules leucémiques. Les produits à examiner ont été administrés aux groupes "essais", par voie intraveineuse, un jour après l'inoculation de la leucémie. La mortalité des animaux des groupes "essais", et "contrôles" a été enregistrée pendant 60 jours après l'inoculation. Dans le Tableau II, est indiqué le nombre de survivants consignés à l'issue de 30 jours et 60 jours d'observation. Après 60 jours d'observation, les animaux ayant survécus ont été considérés en rémission à long terme. Le Tableau III indique les valeurs du pourcentage de la durée de vie moyenne (T.M.S.) des groupes essais T sur la vie moyenne du groupe contrôle C non traité. Des valeurs de T/C (TMS) supérieures à 125% sont significatives d'une activité antitumorale. Le nombre des astérisques indiqué à chaque valeur correspond au nombre des survivants 60 jours après l'inoculation.

Comme les résultats des Tableaux II et III le démontrent, les composés de l'invention possèdent une meilleure activité antitumorale par rapport aux composés de référence vinblastine et vintryptol.

TABLEAU II

| PRODUITS | DOSE mg/kg | SURVIVANTS | | | |
|---|---|---|---|---|---|
| | | MALES | | FEMELLES | |
| | | 30 JOURS | 60 JOURS | 30 JOURS | 60 JOURS |
| VINBLASTINE | 3,0 | | | 1/6 | 1/6 |
| | 5,0 | 1/6 | 1/6 | | |
| VINTRYPTOL | 80,0 | 1/6 | 0/6 | | |
| | 100,0 | 1/6 | 1/6 | 1/6 | 1/6 |
| EXEMPLE 1 (Mélange de diastéréo-isomères) | 0,25 | 2/6 | 2/6 | | |
| | 0,4 | 3/6 | 1/6 | 3/6 | 3/6 |
| | 0,6 | 3/6 | 3/6 | 4/6 | 4/6 |
| EXEMPLE 2 (Mélange de diastéréo-isomères) | 0,4 | | | 1/6 | 1/6 |
| | 0,5 | 5/6 | 3/6 | 1/6 | 1/6 |
| | 0,6 | | | 2/6 | 2/6 |
| EXEMPLE 5 (Mélange de diastéréo-isomères) | 1,0 | 1/6 | 1/6 | | |
| EXEMPLE 6 (Mélange de diastéréo-isomères) | 0,1 | | | 1/6 | 1/6 |
| EXEMPLE 7 | 30 | 3/6 | 1/6 | 1/6 | 1/6 |
| | 40 | 6/6 | 5/6 | 2/6 | 2/6 |
| | 50 | | | 3/6 | 3/6 |
| EXEMPLE 10 | 3 | 1/6 | 1/6 | | |

TABLEAU III

| PRODUITS | DOSE mg/kg | ANIMAUX | |
|---|---|---|---|
| | | *MALES* | *FEMELLES* |
| VINTRYPTOL | 40<br>60<br>80<br>100 | 167<br>174<br>240<br>246 * | 180<br>208<br>215 * |
| VINBLASTINE | 2<br>3<br>5 | 176<br>187<br>201 * | 189 *<br>192 |
| EXEMPLE 1 (Mélange de diastéréo-isomères) | 0,25<br>0,4<br>0,6 | 298 **<br>332 *<br>395 *** | 378 ***<br>395 **** |
| EXEMPLE 2 (Mélange de diastéréo-isomères) | 0,5<br>0,6 | 428 *** | 233 *<br>324 ** |
| EXEMPLE 5 (Mélange de diastéréo-isomères) | 1,0 | 278 * | |
| EXEMPLE 6 (Mélange de diastéréo-isomères) | 0,1 | | 184 * |
| EXEMPLE 7 | 30<br>40<br>50 | 321 *<br>468 ***** | 283<br>350 **<br>416 *** |
| EXEMPLE 10 | 3 | 239 * | |

## EXEMPLE 14

### Activité antitumorale sur la leucémie L 1210 chez la souris

Deux groupes de souris (n=6), de souche $B_6D_2F_1$, de même sexe et âge, ont reçu, par voie intrapéritionéale, 0,4 ml de sérum physiologique contenant en suspension $10^5$ de cellules leucémiques (L1210). Un jour après l'inoculation de la leucémie, les produits à examiner ont été administrés aux groupes "essais" par voie intrapéritonéale. La mortalité a été enregistrée dans chaque expérience pendant 60 jours après l'inoculation. Pour chaque essai, le nombre des survivants consignés à l'issue des 60 jours et le pourcentage de la durée de vie moyenne des groupes essais sur la vie moyenne du groupe contrôle non traité T/C TMS sont rassemblés dans le Tableau IV. Les résultats obtenus lors de cette étude démontrent également la supériorité pharmacologique des composés de l'invention.

**TABLEAU IV**

| PRODUITS | DOSE mg/kg | T/C TMS | SURVIVANTS |
|---|---|---|---|
| VINBLASTINE | 3 | 151 | 0/6 |
| VINTRYPTOL | 40<br>60<br>100 | 101<br>112<br>108 | 0/6<br>0/6<br>0/6 |
| EXEMPLE 2 (Mélange de diastéréo-isomères) | 0,5 | 306 | 2/6 |
| EXEMPLE 5 (Mélange de diastéréo-isomères) | 1,0 | 151 | 0/6 |
| EXEMPLE 7 | 80 | 242 | 1/6 |

**PREPARATION PHARMACEUTIQUE**

**EXEMPLE 15**

**Poudre lyophilisée pour préparation injectable contenant 0,5 mg de N-(désacétyl-0-4 vinblastinoyl-23) amino-1 méthyl-2 propyl phosphonate de diéthyle.**

N-(désacétyl-0-4 vinblastinoyl-23) amino-1 méthyl-2 propyl phosphonate de diéthyle ........................................ 0,5 mg

Lactose anhydre... .................................... 10 mg

pour un flacon de poudre.

**Revendications**

**Revendications pour les Etats contractants suivants : AT, BE, CH, DE, FR, GB, IT, LI, LU, NL, SE.**

1. Composés de formule I :

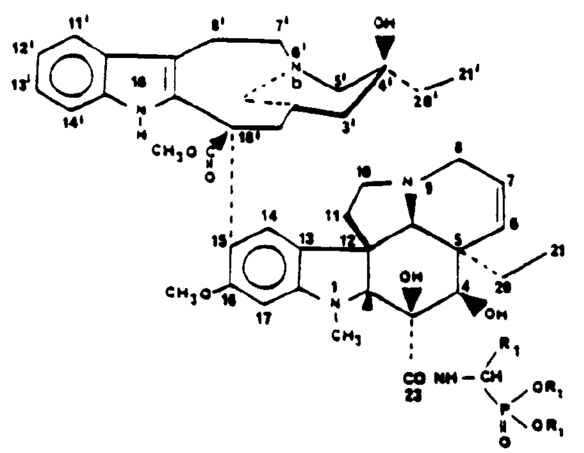

(I)

dans laquelle :

– $R_1$ représente un atome d'hydrogène, un radical alkyle linéaire ou ramifié renfermant de 1 à 6 atomes de carbone, un radical alkylène linéaire ou ramifié renfermant de 1 à 6 atomes de carbone, un radical arylalkyle de 7 à 10 atomes de carbone pouvant porter comme substituant sur le cycle aromatique un atome d'halogène un radical hydroxyle ou un radical alkyle ou alcoxy renfermant chacun de 1 à 5 atomes de carbone, un radical indolyl-2 méthyle, un radical imidazolyl-4 méthyle, ou un radical alcoxycarbonylméthyle renfermant de 3 à 11 atomes de carbone,

– $R_2$ et $R_3$ identiques ou différents représentent chacun un radical alkyle linéaire ou ramifié renfermant de 1 à 4 atomes de carbone,

sous forme de mélange de diastéréo-isomères ou d'isomères purs, leurs $N_{b'}$-oxydes, et leurs sels d'addition à un acide minéral ou organique pharmaceutiquement acceptable.

2. Composés de formule I, selon la revendication 1 dans laquelle :

– $R_1$ représente un radical alkylène linéaire ou ramifié renfermant de 1 à 6 atomes de carbone et

– $R_2$ et $R_3$ identiques représentent chacun un radical alkyle linéaire renfermant de 1 à 4 atomes de carbone, sous forme de mélange de diastéréo-isomères ou d'isomères purs, leurs $N_{b'}$-oxydes et leurs sels d'addition à un acide minéral ou organique pharmaceutiquement acceptable.

3. Le N-(désacétyl-0-4 vinblastinoyl-23) amino-1 méthyl-2 propyl phosphonate de diéthyle, composé répondant à la formule I selon la revendication 1, sous forme de mélange de diastéréo-isomères ou d'isomères purs, et ses sels d'addition à un acide minéral ou organique pharmaceutiquement acceptable.

4. Le N-(désacétyl-0-4 vinblastinoyl-23) amino-1 éthyl phosphonate de diéthyle, composé répondant à la formule I selon la revendication 1, sous forme de mélange de diastéréo-isomères ou d'isomères purs, et ses sels d'addition à un acide minéral ou organique pharmaceutiquement acceptable.

5. Procédé de préparation de composés de formule I selon la revendication 1, caractérisé en ce que l'on fait réagir une amine, sous forme racémique ou optiquement pure, de formule générale II :

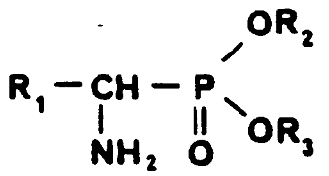

(II)

dans laquelle la définition de $R_1$, $R_2$ et $R_3$ demeure celle définie précédemment pour la formule générale I, selon la revendication 1, à température ambiante, dans un solvant organique dérivé d'alkyles halogénés inférieurs,

avec le descarbométhoxy-3 désacétyl-0-4 vinblastine carboxazide-3, composé de formule III,

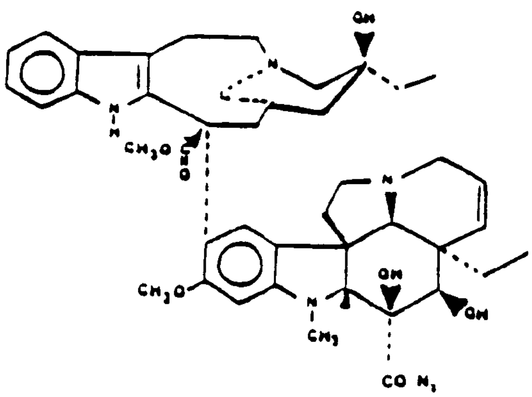

(III)

pour former respectivement les composés de formule générale I selon la revendication 1, sous forme de mélange de diastéréo-isomères ou isomères purs,

lesquels ensuite on peut :

– soit salifier avec un acide minéral ou organique pharmaceutiquement acceptable;

– soit transformer aux $N_{b'}$-oxides correspondants à l'aide d'un solvant organique basique d'oxygène.

6. Composition pharmaceutique contenant comme principe actif un composé selon l'une quelconque des revendications 1 à 4, en association ou en mélange avec un excipient ou un véhicule inerte, non toxique, pharmaceutiquement acceptable.

7. Composition pharmaceutique selon la revendication 6 renfermant le principe actif à la dose de 0,1 à 100 mg.

8. Composition pharmaceutique selon les revendications 6 et 7 renfermant comme principe actif au moins un composé selon les revendications 1 à 4 utilisable pour le traitement des maladies néoplastiques chez l'être vivant.

**Revendications pour les Etats contractants suivants : ES, GR.**

1. Procédé de préparation des composés de formule I :

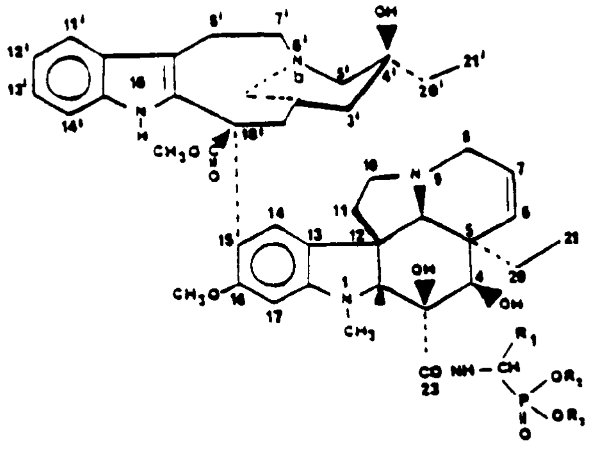

(I)

dans laquelle :

– $R_1$ représente un atome d'hydrogène, un radical alkyle linéaire ou ramifié renfermant de 1 à 6 atomes de carbone, un radical alkylène linéaire ou ramifié renfermant de 1 à 6 atomes de carbone, un radical arylalkyle de 7 à 10 atomes de carbone pouvant porter comme substituant sur le cycle aromatique un atome d'halogène un radical hydroxyle ou un radical alkyle ou alcoxy renfermant chacun de 1 à 5 atomes de carbone, un radical indolyl-2 méthyle, un radical imidazolyl-4 méthyle, ou un radical alcoxycarbonylméthyle renfermant de 3 à 11 atomes de carbone,

– $R_2$ et $R_3$ identiques ou différents représentent chacun un radical alkyle linéaire ou ramifié renfermant de 1 à 4 atomes de carbone,

sous forme de mélange de diastéréo-isomères ou d'isomères purs, leurs $N_{b'}$-oxydes, et leurs sels d'addition à un acide minéral ou organique pharmaceutiquement acceptable, caractérisé en ce que l'on fait réagir une amine, sous forme racémique ou optiquement pure, de formule générale II :

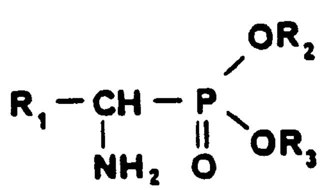

(II)

dans laquelle la définition de $R_1$, $R_2$ et $R_3$ demeure celle définie précédemment pour la formule générale I, à température ambiante, dans un solvant organique dérivé d'alkyles halogénés inférieurs,

avec le descarbométhoxy-3 désacétyl-0-4 vinblastine carboxazide-3, composé de formule III,

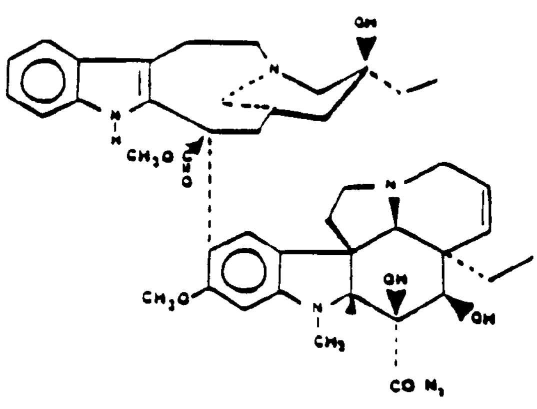

(III)

pour former respectivement les composés de formule générale I sous forme de mélange de diastéréoisomères ou isomères purs,

lesquels ensuite on peut :

– soit salifier avec un acide minéral ou organique pharmaceutiquement acceptable;

– soit transformer aux $N_{b'}$-oxydes correspondants à l'aide d'un solvant organique basique saturé d'oxygène.

## Claims

### Claims for the following Contracting States : AT, BE, CH, DE, FR, GB, IT, LU, NL, SE

1. Compounds of formula I:

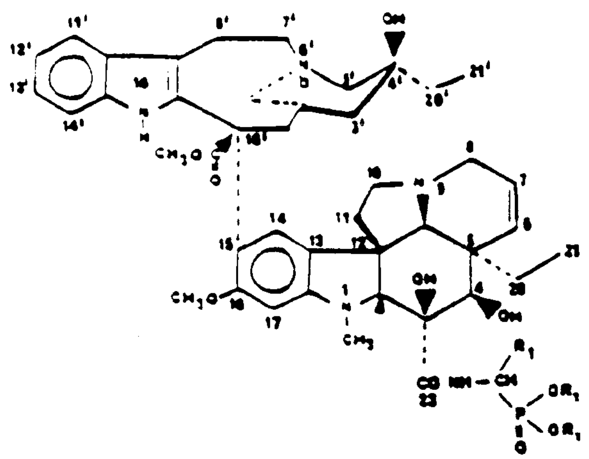

(I)

in which :

– $R_1$ represents a hydrogen atom, a linear or branched alkyl radical containing from 1 to 6 carbon atoms, a linear or branched alkylene radical containing from 1 to 6 carbon atoms, an aralkyl radical containing from 7 to 10 carbon atoms that may carry as substituent on the aromatic ring a halogen atom, a hydroxy radical, or an alkyl or alkoxy radical each containing from 1 to 5 carbon atoms; a 2-indolylmethyl radical, a 4-imidazolylmethyl radical, or an alkoxycarbonylmethyl radical containing from 3 to 11 carbon atoms,

– each of $R_2$ and $R_3$, which are the same or different, represents a linear or branched alkyl radical containing from 1 to 4 carbon atoms,

in the form of a mixture of diastereoisomers or pure isomers, their $N_{b'}$-oxides, and their addition salts with a pharmaceutically acceptable mineral or organic acid.

2. Compounds of formula I according to claim 1 in which:

– $R_1$ represents a linear or branched alkylene radical containing from 1 to 6 carbon atoms, and

– each of $R_2$ and $R_3$, which are the same, represents a linear alkyl radical containing from 1 to 4 carbon atoms,

in the form of a mixture of diastereoisomers or pure isomers, their $N_{b'}$-oxides, and their addition salts with a pharmaceutically acceptable mineral or organic acid.

3. 1-[N-(4-0-deacetyl-23-vinblastinoyl)amino]-2-methylpropyl diethyl phosphonate, a compound corresponding to formula I according to claim 1, in the form of a mixture of diastereoisomers or pure isomers, and its addition salts with a pharmaceutically acceptable mineral or organic acid.

4. 1-[N-(4-0-deacetyl-23-vinblastinoy1)amino]-ethyl diethyl phosphonate, a compound corresponding to formula I according to claim 1, in the form of a mixture of diastereoisomers or pure isomers, arid its addition salts with a pharmaceutically acceptable mineral or organic acid.

5. Process for the preparation of compounds of formula I according to claim 1, characterised in that an amine, in racemic or optically pure form, of the general formula II:

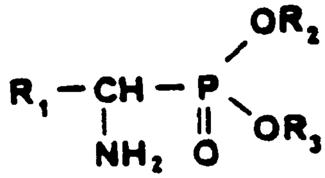

(II),

in which $R_1$, $R_2$ and $R_3$ are as defined above for the general formula I according to claim 1, is reacted at room temperature, in an organic solvent derived from halogenated lower alkyls,

with 3-demethoxycarbonyl-4-0-deacetylvinblastine 3-carboxazide, the compound of formula III

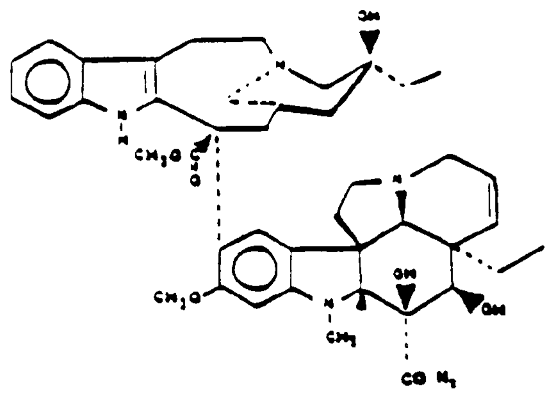

(III)

to form the compounds of the general formula I according to claim 1 in the form of a mixture of diastereoisomers or pure isomers, respectively,

which may then:

– either be converted into a salt with a pharmaceutically acceptable mineral or organic acid; or

– be converted into the corresponding $N_{b'}$-oxides with the aid of a basic organic solvent saturated with oxygen.

6. Pharmaceutical composition that contains as active ingredient a compound according to any one of claims 1 to 4, in association or in admixture with a pharmaceutically acceptable inert, non-toxic excipient or vehicle.

7. Pharmaceutical composition according to claim 6 that contains the active ingredient in an amount of from 0.1 to 100 mg.

8. Pharmaceutical composition according to claims 6 and 7 that contains as active ingredient at least one compound according to claims 1 to 4 and that is usable for the treatment of neoplastic disorders in living beings.

**Claims for the following Contracting States : ES, GR**

1. Process for the preparation of compounds of formula I:

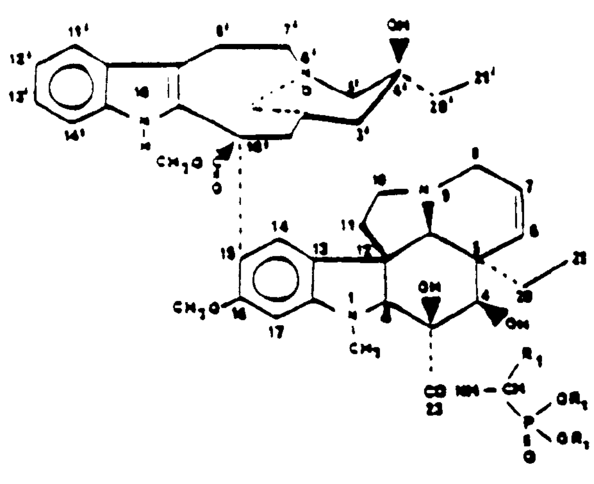

(I)

in which :

– $R_1$ represents a hydrogen atom, a linear or branched alkyl radical containing from 1 to 6 carbon atoms, a linear or branched alkylene radical containing from 1 to 6 carbon atoms, an aralkyl radical containing from 7 to 10 carbon atoms that may carry as substituent on the aromatic ring a halogen atom, a hydroxy radical, or an alkyl or alkoxy radical each containing from 1 to 5 carbon atoms; a 2-indolylmethyl radical, a 4-imidazolylmethyl radical, or an alkoxycarbonylmethyl radical containing from 3 to 11 carbon atoms,

– each of $R_2$ and $R_3$, which are the same or different, represents a linear or branched alkyl radical containing from 1 to 4 carbon atoms,

in the form of a mixture of diastereoisomers or pure isomers, their $N_{b'}$-oxides, and their addition salts with a pharmaceutically acceptable mineral or organic acid,

characterised in that an amine, in racemic or optically pure form, of the general formula II:

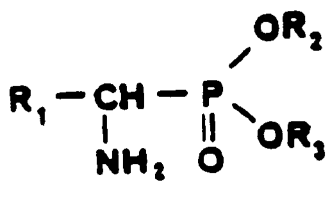

(II),

in which $R_1$, $R_2$ and $R_3$ are as defined above for the general formula I, is reacted at room temperature, in an organic solvent derived from halogenated lower alkyls,

with 3-demethoxycarbonyl-4-0-deacetylvinblastine 3-carboxazide, the compound of formula III

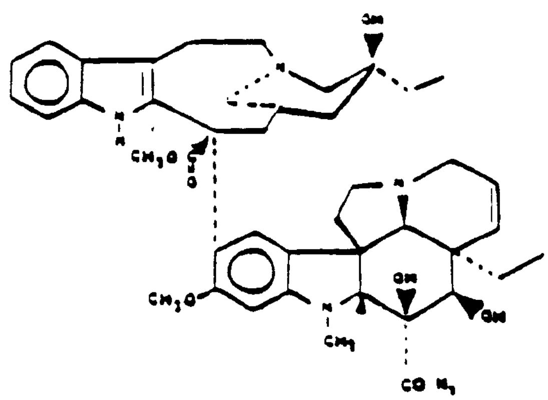

(III)

to form the compounds of the general formula I in the form of a mixture of diastereoisomers or pure isomers, respectively,

which may then:

– either be converted into a salt with a pharmaceutically acceptable mineral or organic acid; or

– be converted into the corresponding $N_{b'}$-oxides with the aid of a basic organic solvent saturated with oxygen.

## Patentansprüche

### Patentansprüche für folgende Vertragsstaaten : AT, BE, CH, DE, FR, GB, IT, LI, LU, NL, SE

1. Verbindungen der Formel I

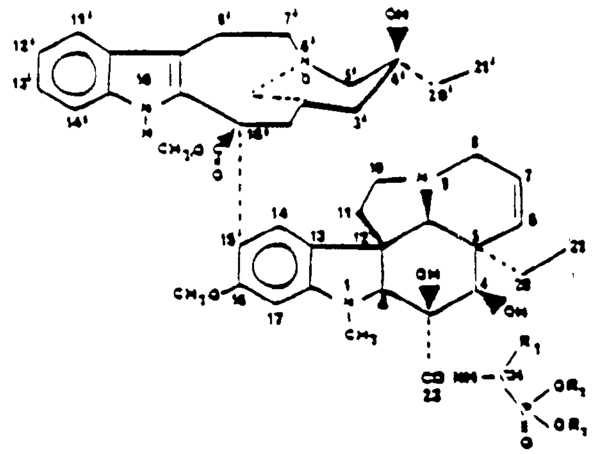

(I)

in der

– $R_1$ ein Wasserstoffatom, eine geradkettige oder verzweigte Alkylgruppe mit 1 bis 6 Kohlenstoffatomen, eine geradkettige oder verzweigte Alkylengruppe mit 1 bis 6 Kohlenstoffatomen, eine Aralkylgruppe mit 7 bis 10 Kohlenstoffato. men, welche als Substituent am aromatischen Ring ein Halogenatom, eine Hydroxylgruppe oder eine Alkyl- oder Alkoxygruppe mit 1 bis 5 Kohlenstoffatomen aufweisen kann, eine Indol-2-yl-methylgruppe, eine Imidazol-4-yl-methylgruppe oder eine Alkoxycarbonylmethylgruppe mit 3 bis 11 Kohlenstoffatomen, und

– $R_2$ und $R_3$, die gleichartig oder verschieden sein können, jeweils gerad° kettige oder verzweigte Alkylgruppen mit 1 bis 4 Kohlenstoffatomen bedeuten, in Form einer Mischung der Diastereoisomeren oder der reinen Isomeren, deren $N_{b'}$-Oxide und deren Additionssalze mit einer anorganischen oder organischen, pharmazeutisch annehmbaren Säure.

2. Verbindungen der Formel I gemäß Anspruch 1, worin:

– $R_1$ eine geradkettige oder verzweigte Alkylengruppe mit 1 bis 6 Kohlenstoffatomen und

– $R_2$ und $R_3$ gleichartig sind und geradkettige Alkylgruppen mit 1 bis 4 Kohlenstoffatomen bedeuten,

in Form einer Mischung der Diastereoisomeren oder der reinen Isomeren, deren $N_{b'}$-Oxide und deren Additionssalze mit einer anorganischen oder organischen, pharmazeutisch annehmbaren Säure.

3. Diethyl-N-(4-0-desacetyl-23-vinblastinoyl)-1-amino-2-methyl-propylphosphonat der Formel I nach Anspruch 1, in Form einer Mischung der Diastereoisomeren oder der reinen Isomeren, und deren Additionssalze mit einer anorganischen oder organischen, pharmazeutisch annehmbaren Säure.

4. Diethyl-N-(4-0-desacetyl-23-vinblastinoyl)-1-amino-ethyl-phosphonat der Formel I nach Anspruch 1, in Form einer Mischung der Diastereoisomeren oder der reinen Isomeren und deren Additionssalze mit einer anorganischen oder organischen, pharmazeutisch annehmbaren Säure.

5. Verfahren zur Herstellung der Verbindungen der Formel I nach Anspruch 1, **dadurch gekennzeichnet**, daß man ein racemisches oder optisch reines Amin der allgemeinen Formel II

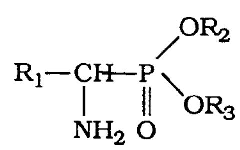

(II)

in der $R_1$, $R_2$ und $R_3$ die in Anspruch 1 bezüglich der allgemeinen Formel I angegebenen Bedeutungen besitzen, bei Raumtemperatur in einem von niedrigmolekularen Alkylhalogeniden abgeleiteten organischen Lösungsmittel mit 3-Descarbomethoxy-4-0-desacetyl-vinblastin-3-carboxazid der Formel III

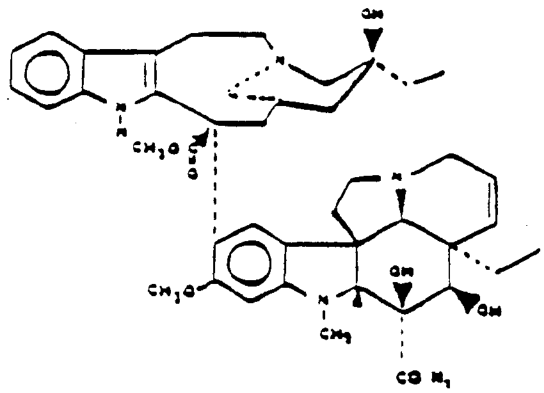

(III)

umsetzt,

zur Bildung der Verbindung der allgemeinen Formel I in Form einer Mischung der Diastereoisomeren oder der reinen Isomeren, welche man anschließend

– entweder mit einer anorganischen oder organischen, pharmazeutisch annehmbaren Säure in ein Salz überführen;

– oder mit einem mit Sauerstoff gesättigten, basischen organischen. Lösungsmittel in die entsprechenden $N_{b'}$-Oxide umwandeln kann.

6. Pharmazeutische Zubereitung enthaltend als Wirkstoff eine Verbindung nach einem der Ansprüche I bis 4 in Kombination oder in Mischung mit einem inerten, nichttoxischen, pharmazeutisch annehmbaren Trägermaterial und/ oder Bindemittel.

7. Pharmazeutische Zubereitung nach Anspruch 6, enthaltend den Wirkstoff in einer Dosis von 0,1 bis 100 mg.

8. Pharmazeutische Zubereitung nach den Ansprüchen 6 und 7, enthaltend als Wirkstoff mindestens eine Verbindung nach einem der Ansprüche 1 bis 4 zur Verwendung bei der Behandlung von neoplastischen Erkrankungen am lebenden Organismus.

**Patentansprüche für folgende Vertragsstaaten : ES, GR**

1. Verfahren zur Herstellung der Verbindungen der allgemeinen Formel I

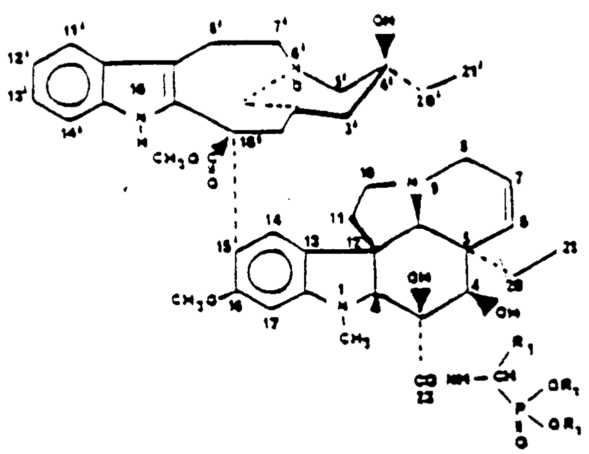

(I)

in der

– $R_1$ ein Wasserstoffatom, eine geradkettige oder verzweigte Alkylgruppe mit 1 bis 6 Kohlenstoffatomen, eine geradkettige oder verzweigte Alkylengruppe mit 1 bis 6 Kohlenstoffatomen, eine Aralkylgruppe mit 7 bis 10 Kohlenstoffatomen, welche als Substituent am aromatischen Ring ein Halogenatom, eine Hydroxylgruppe oder eine Alkyl- oder Alkoxygruppe mit 1 bis 5 Kohlenstoffatomen aufweisen kann, eine Indol-2-yl-methylgruppe, eine Imidazol-4-yl-methylgruppe oder eine Alkoxycarbonylmethylgruppe mit 3 bis 11 Kohlenstoffatomen, und

– $R_2$ und $R_3$, die gleichartig oder verschieden sein können, jeweils geradkettige oder verzweigte Alkylgruppen mit 1 bis 4 Kohlenstoffatomen bedeuten, in Form einer Mischung der Diastereoisomeren oder der reinen Isomeren, deren $N_{b'}$-Oxide und deren Additionssalze mit einer anorganischen oder organischen, pharmazeutisch annehmbaren Säure,

**dadurch gekennzeichnet**, daß man ein racemisches oder optisch reines Amin der allgemeinen Formel II

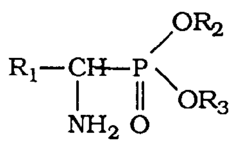

(II)

in der $R_1$, $R_2$ und $R_3$ die oben bezüglich der allgemeinen Formel I angegebenen Bedeutungen besitzen, bei Raumtemperatur in einem von niedrigmolekularen Alkylhalogeniden abgeleiteten organischen Lösungsmittel mit 3-Descarbomethoxy-4-0-desacetyl-vinblastin-3-carboxazid der Formel III

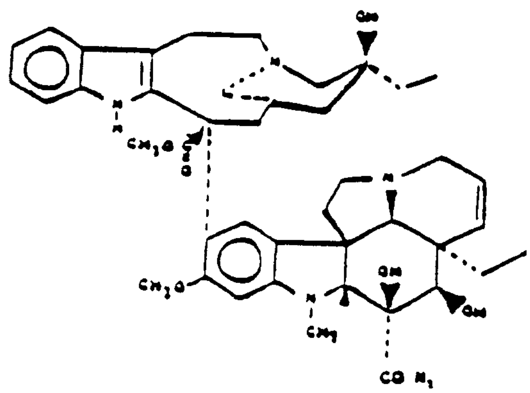

(III)

umsetzt,

zur Bildung der Verbindung der allgemeinen Formel I in Form einer Mischung der Diastereoisomeren oder der reinen Isomeren, welche man anschließend

– entweder mit einer anorganischen oder organischen, pharmazeutisch annehmbaren Säure in ein Salz überführen;

– oder mit einem mit Sauerstoff gesättigten, basischen organischen Lö-sungsmittel in die entsprechenden $N_{b'}$-Oxide umwandeln kann.